# EUROPEAN PATENT APPLICATION

(11) **EP 2 609 911 A1**
(43) Date of publication of application: **03.07.2013**
(21) Application number: 12199670.6
(22) Date of filing: 28.12.2012
(51) Int. Cl.: A61K 9/00, A61K 47/32

(54) **A novel process for preparing orally disintegrating flurbiprofen formulations**

(30) Priority: 29.12.2011 TR 201113209
(71) Applicant: Sanovel Ilac Sanayi ve Ticaret A.S., 34460 Istanbul (TR)
(72) Inventor: Cifter, Ümit, 34460 Istanbul (TR); Türkyilmaz, Ali, 34460 Istanbul (TR); Pehilvan Akalin, Nur, 34460 Istanbul (TR); Önder, Ramazan, 34460 Istanbul (TR)
(74) Representative: Sevinç, Erkan

(57) **Abstract**

The present invention is related to a novel process for preparing flurbiprofen orally disintegrating formulations, more specifically the present invention is associated with the decrease of potential side effects of flurbiprofen that may arise in esophagus and stomach by prevention any irritation related to flurbiprofen during its passage through esophagus by ensuring the release of it in stomach.

## Description

### Technical Aspect

The present invention is related to a novel process for preparing flurbiprofen orally disintegrating formulation, more specifically the present invention is associated with the decrease of potential side effects of flurbiprofen that may arise in esophagus and stomach by prevention any irritation related to flurbiprofen during its passage through esophagus by ensuring the release of it in stomach.

### Background of the Invention

Flurbiprofen is a well known, propionic acid derivative, also known as NSAID (non-steroidal anti-inflammatory drug), with the analgesic and anti-inflammatory activities it possesses. It is used in muscle-skeletal and joint disorders such as ankylosing spondylitis, osteoarthritis and rheumatoid arthritis, in soft-tissue disorders such as sprains and strains and for postoperative pains and mild to moderate pain including dysmenorrhoea and migraine. Its chemical structure is illustrated with Formula I given below.

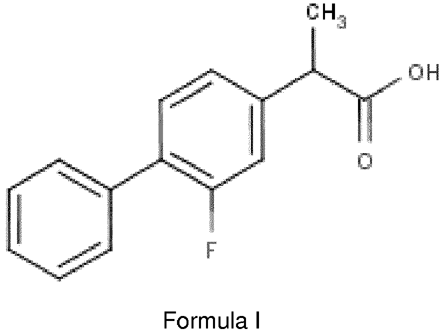

Flurbiprofen is mostly administrated orally. One disadvantage of the oral administration of flurbiprofen comprising compositions is that the patient is likely to experience unpleasant side effects, including irritation related to its passage through esophagus and gastrointestinal irritation.

Flurbiprofen is also used as lozenges in the symptomatic relief of sore throat, but because of irritation related to flurbiprofen through esophagus there may arise other dosage forms such as orally disintegrating formulations.

An additional problem may arise from oral administration of flurbiprofen that it has a bitter taste and an irritant drug when formulated as an orally disintegrating dosage form. Therefore a need arise to formulate an orally disintegrating formulation of flurbiprofen which has a pleasant taste and not causing any irritation during its passage through esophagus by ensuring the release of it in stomach.

Orally disintegrating formulations are becoming an increasingly important issue in the area of better patient compliance comparative to the conventional solid dosage forms for oral administration such as capsules and tablets, which are the most commonly used. In particular pediatric and geriatric patients often experience difficulties in swallowing solid dosage forms. Besides, conventional solid dosage forms are not suitable for bedridden or busy and travelling patients, in case the patient may not have easy access to water. Thus, orally disintegrating compositions represent an alternative for such patients and provide for a better patient compliance with recommended pharmaceutical therapies.

It is known that, to develop orally disintegrating compositions are difficult because of several different reasons. A satisfied orally disintegrating dosage form needs to meet number of requirements. Firstly, it has to disintegrate in the oral cavity rapidly. Moreover, a premature release in the mouth could also lead to problems due to the often unpleasant taste of the active ingredient. Besides, these compositions should be very porous and should not be very hard. These porous compositions tend to be very sensitive to humidity. As a consequence, they may have some stability problems. Finally, any orally disintegrating composition with suitable organoleptic and pharmacokinetic properties must also be manufactured at commercially useful rates and yields and with more simple methods.

To fulfill all these requirements the formulation for a specific drug needs to be adapted in particular by a careful selection of the excipients used. However, the excipients selected may lead to formulations which are not bioavailable to the corresponding conventional dosage forms. Thus, they have to be chosen very carefully. Additionally, precautions have to be taken at the preparation, packaging, handling and storing of the finished dosage forms of orally disintegrating compositions since they tend to be both hygroscopic and friable.

Thus, various technologies have been developed which enable the preparation of compositions that disintegrate quickly in the oral cavity. These technologies are including spray drying, freeze drying and floss formation. However, all these technologies have their own limitations and have very complicated manufacturing processes.

The spray drying technique involves spraying the drug and excipients into a chamber maintained at a high temperature. As a result this technique is not suitable for application to thermolabile drugs. Additionally, the spray drying technology leads only to a very poor output and is very expensive.

Freeze drying on a large scale has not been found to be very effective. Moreover, it has limitations due to factors such as time, costly equipment and processing conditions. Besides, the Zydis^{®} tablets prepared by this technique are so fragile that the formation of the matrix material has to take place in a specific container. Tablets manufactured by this technology require a special type of packaging and careful handling during dispensing and administration to the patients, since they are prone to breakage. Such US Patents which disclose these formulations are US 4 642 903, 5 188 825, 5 631 023, 5 827 541 and 5 976 577.

The floss formation technique includes compressing micro- particles of a drug and a cotton candy-like fibrous saccharide matrix, such as sucrose, dextrose, lactose and fructose. It is also known as Flash Dose technology (Fuisz) and requires specific equipment for making the specific matrix, which is sensitive to moisture, and generally results in tablets of high friability.

Finally, many of these techniques have proved to be successful only for specific drugs and are often not transferable to other active ingredients such as flurbiprofen.

Thus, a need rises for orally disintegrating tablet formulations of flurbiprofen or a pharmaceutically acceptable salt thereof and a process for preparing such formulation which overcomes the above described problems in prior art and having additive advantages over its unpleasant taste and irritation problems. Further advantages and embodiments of the present invention will become apparent from the following description.

### Summary of the Invention

The present invention relates to an improved process for preparing orally disintegrating formulations of flurbiprofen or a pharmaceutically acceptable salt thereof which overcomes the above described problems with using adequate excipients and which further provide the advantageous property of allowing the active medicament to disintegrate rapidly in the oral cavity by preventing potential side effects of flurbiprofen such as irritation and unpleasant taste that may arise in mouth and esophagus during its passage through it, by ensuring the release of flurbiprofen in stomach.

Another object of the present invention is to provide a simple, cost-effective and time saving process for the preparation of orally disintegrating tablet formulation of flurbiprofen or a pharmaceutically acceptable salt thereof.

Yet another object of the present invention is to provide an orally disintegrating tablet formulation of flurbiprofen or a pharmaceutically acceptable salt thereof which has good mechanical strength enough to be processed in high speed tableting machines and shipped in low cost packages.

A further object of the present invention is to provide bioavailable and stable orally disintegrating tablet formulation of flurbiprofen or a pharmaceutically acceptable salt thereof throughout the shelf-life.

In a preferred embodiment according to the present invention, said novelty and improvement is realized with a process for preparing orally disintegrating flurbiprofen or a pharmaceutically acceptable salt therof comprising the following steps;
a) blending 0.10 % to 5,0 % by weight of at least one polymeric binder which is selected from group consisting of polyvinylpyrrolidone, hydrocypropyl methylcellulose, hydroxypropyl cellulose, hydroxyethyl cellulose, methyl cellulose, ethyl cellulose, cellulose acetate, cellulose acetate phthalate, cellulose acetate trimellitate, hydroxypropyl methylcellulose phthalate, carboxymethylcellulose sodium, polyvinyl acetate, polyvinylacetate phthalate and vinylacetate crotonic acid copolymer and combinations thereof with flurbiprofen or a pharmaceutically acceptable salt therof,
b) granulating the mixture with alcohol and/or water,
c) coating the granule with a surfactant free mixture which is selected from a group comprising polyvinyl alcohol or polyvinyl alcohol copolymers in a weight ratio between 0.50 to 5.0 % by weight.
d) adding other excipients to coated granule and mixing them,
e) adding sodium stearyl fumarate to this mixture and blending them until obtaining a homogenous powder mixture,

According to a preferred embodiment, the polymeric binder is polyvinylpyrrolidone in a weight ratio of 2.0 % by weight.

According to a preferred embodiment, the weight ratio of the surfactant free mixture of polyvinyl alcohol or polyvinyl alcohol copolymers is 2.0 % by weight.

According to a preferred embodiment, the weight ratio of the sodium stearyl fumarate is between 0.10 to 5.0 %, preferably is between 0.50 to 3.0 % by weight, more preferably it is 1.50 % by weight.

According to a preferredembodiment, the pharmaceutically acceptable salt of flurbiprofen is sodium salt.

According to a preferred embodiment, the other excipients are selected from the group comprising of crospovidone, mannitol, microcrystalline cellulose, mixture of microcrystalline cellulose and guar gum, sucralose and orange flavor.

According to another preferred embodiment, the process for preparing orally disintegrating formulations of flurbiprofen or a pharmaceutically acceptable salt therof is comprising the following steps;
a) blending 0.10 % to 5.0 % by weight with polyvinylpyrrolidone with flurbiprofen sodium salt,
b) granulating the mixture with alcohol and/or water,
c) drying and sieving the granule,
d) coating the sieved granule with a surfactant free mixture which is polyvinyl alcohol or polyvinyl alcohol copolymers in a weight ratio between 2.0 % by weight.
e) adding crospovidone, mannitol, microcrystalline cellulose, mixture of microcrystalline cellulose and guar gum, sucralose and orange flavor to coated granule and mixing them,
f) adding sodium stearyl fumarate to this mixture and blending them until obtaining a homogenous powder mixture,
g) compressing the blended mixture to form tablets.

According to the preferred embodiment, the weight ratio of crospovidone and sodium stearyl fumarate is between 25:1 to 1:5 by weight of the total composition, preferably between 15:1 to 1:1 (w/w), more preferably it is 10:1 (w/w).

According to the preferred embodiment, wherein the weight ratio of mannitol to microcrystalline cellulose is in the range of between 10:1 to 1:10 (w/w), preferably it is 5:1 to 1:5 (w/w), more preferably it is 2:1 to 1:2 (w/w).

According to a further preferred embodiment, orally disintegrating tablet formulation of flurbiprofen or a pharmaceutically acceptable salt thereof consists of,
a) 25.0 % by weight flurbiprofen pharmaceutically acceptable salt thereof
b) 2.0 % polyvinylpyrrolidone
c) 2.0 % polyvinylalcohol
d) 1.50 % sodium stearyl fumarate
e) 15.0 % crospovidone
f) 30.3 %mannitol
g) 18.0 % microcrystalline cellulose
h) 5.0 % mixture of microcrystalline cellulose and guar gum
i) 1.0 % orange flavor
j) 0.20 % sucralose

According to this embodiment, the pharmaceutically acceptable salt of flurbiprofen is sodium salt.

According to the further embodiment, orally disintegrating tablets disintegrate in oral cavity in less than 60 seconds, preferably in less than 30 seconds.

According to the further embodiment, the hardness of the tablets are between 5 N to 100 N, preferably it is between 20 N to 50 N, and the friability of the tablets are less than 1.0%, preferably less than 0.50%.

### Detailed Description of the invention

It is known that flurbiprofen has an unpleasant taste and more importantly it has an irritant effect, to prevent the unpleasant taste and the irritant effect of flurbiprofen in mouth and esophagus, a novel and improved process method is now found which is also not very complicated as the known ones in prior art.

According to this embodiment the process can be described as granulating the flurbiprofen with polyvinylpyrrolidone and then granulated particles are coated with polyvinyl alcohol which provides the disintegration of the tablets less than 30 seconds in the oral cavity as in the form of coated granules and they pass trough the esophagus without releasing the flurbiprofen in the mouth and directly reaches to the gastrointestinal system. This provides the effect that prepared dispersable flurbiprofen particles should not be released from the coated granulate preferably in 1 minute, more preferably 30 seconds in the oral cavity. At the end of 1 minute, the release of not more than 10% w/v of flurbiprofen is permissible and this effect is achieved by the weight ratio of polyvinyl alcohol from 0.5 to 5.0 % by weight. Accordingly this protecting effect of the polyvinyl alcohol does not have the retartand effect on dissolution of the coated granules in the gastrointestinal system. Thus, flurbiprofen dissolves very quickly in the absorbtion area and provide the oral bioavailabilty sufficiently at the desired level. As a result of this, the unpleasant taste and more importantly the irritant effect of flurbiprofen therefore is prevented surprisingly with the coated granules of flurbiprofen according to the present invention.

According to this embodiment when the dissolution assay is performed with the formulation of the present invention in 900 ml, pH: 7.2 KH2PO4 buffer; 50 rpm; USP paddle method; 37°C ± 0.5°C at least 85 % of flurbiprofen is dissolved in 15 minutes.

Another object of the present invention is granulating the flurbiprofen with polyvinyl pyrrolidone (PVP) and this provide the effect that the granules prepared with PVP has better flow properties and can easily be coated with polyvinylalcohol (PVA) or polyvinylalcohol copolymers. Because flurbiprofen is in micron size particle size and therefore it is difficult to coat in fluid bed granulator and it is difficult to compress these granules into the tablets in optimal mechanical strength. It is very important when formulating orally disintegrating tablets to have robust tablets (e.g., low friability, adequate hardness).

Another object of the invention is to provide an orally disintegrating tablet formulation of flurbiprofen or a pharmaceutically acceptable salt thereof which comprise sodium stearyl fumarate instead of magnesium stearate.

In prior art, most commonly used excipient in orally disintegrating tablet formulations with flurbiprofen as lubricant, is magnesium stearat. It is known that magnesium stearate has some disadvantages despite being a good lubricant and because of this it is used in small quantities during drug manufacturing process. Magnesium stearate is practically insoluble in water and because of this hydrophobic characteristic it may retard the disintegration time. Another disadvantage of magnesium stearate is found in manufacturing process which is the blending time. Blending time should be limited. Long blending times can result in the formulation of hydrophobic powder beds that do not disperse easily and overblending can cause compaction problems. Tablet disintegration time increased and crushing strength decreased as the time of blending increased; and magnesium stearate may also increase tablet friability.

Sodium stearyl fumarate is extremely effective lubricant and less hydrophobic than magnesium stearate. It improves the wettebality of the tablet ingredients, resulting in shorter disintegration times. Sodium stearyl fumarate also doesn't have the over blending problems seen with magnesium steare. Besides, sodium stearyl fumarate can perform more than one function in the formulation of this present invention. For example it can function as both lubricant and a stabilizer. This helps the formulation to stay stable throughout the shelf-life.

According to another embodiment, crospovidone, has physical and chemical properties that make it ideal for constituting the appropriate disintegrant for this invention. Because crospovidone particles have a very different appearance from those of the other disintegrants. Crospovidone particles seem to consist of aggregates of smaller particles that are fused together. This aggregation gives crospovidone a spongy, highly porous appearance and it swells very little, yet takes water into its network quite rapidly. This helps crospovidone to dissolve easily and quickly in a little amount of water or saliva and makes its disintegrating rate much faster than other related excipients.

It has surprisingly been found that the specific combination of crospovidone and sodium stearyl fumarate with the active ingredient flurbiprofen or a pharmaceutically acceptable salt thereof within the process which comprise the steps of blending with a polymeric binder, preferably polyvinyl pyrrolidone and forming granules and coating the granules with polyvinyl alcohol results a synergistic effect over the disintegration time and stability. According to this embodiment, the weight ratio of crospovidone to sodium stearyl fumarate is in between 25:1 to 1:1 (w/w), preferably in between 15:1 to 1:1 (w/w), more preferably it is 10:1 (w/w). Accordingly, with the help of these excipients, the formulation disintegrates in oral cavity in less than 60 seconds, preferably in less than 30 seconds.

Another object of the present invention is to develop orally disintegrating compositions having optimal mechanical strength. The present invention addresses this need and discloses formulations that rapidly disintegrate in the oral cavity. These tablet compositions have a pleasant mouth feel and good mechanical strength. These tablets are robust (e.g., low friability, adequate hardness) enough to be processed in high speed tableting machines and shipped in low cost packages, and at the same time retain rapid disintegration or dissolution properties. These orally disintegrating compositions are bioavailable in correspondence with the conventional solid dosage formulations and stable throughout the shelf-life.

It has surprisingly been found that the specific combination of mannitol and microcrystalline cellulose with the active ingredient flurbiprofen or a pharmaceutically acceptable salt thereof within the process which comprise the steps of blending with a polymeric binder, preferably polyvinyl pyrrolidone and forming granules and coating the granules with polyvinyl alcohol or polyvinyl alcohol copolymers results a synergistic effect over the mechanical strength (such as; hardness and friability) of the forrmulation. According to this embodiment, the weight ratio of mannitol to microcrystalline cellulose is in the range of between 10:1 and 1:10 (w/w), preferably it is in the range of between 5:1 and 1:5 (w/w), more preferably it is in the range of between 2:1 to 1:2 (w/w); said amount makes it possible to significantly improve compressibility and reduce friability. Higher quantities may have negative mechanichal strength of the formulation and lower quantities may worsen the disintegration time. According to this object of the present invention, the hardness of the orally disintegrating tablet is between 5 N to 100 N, preferably it is between 20 N to 50 N; and the friabilite of the orally disintegrating tablet is less than 1.0%, preferably less tnah 0.50%.

Suitable sweetners are selected from the group comprising sucralose, fructose, glucose, sorbitol, saccharin, sodium cyclamate, acesulfame-K and the like and mixtures thereof; preferably the sweetner is sucralose. The selection of sucralose as sweetner has lots of advantages such as improving patient compliance. In prior art, it is know that aspartame is used mostly as sweetner but contradictory to the prior art we have found that the effect of sucralose as a sweetner in this formulation, not only helped to improve its taste but also increased the efficacy and the conveniency of the formulation because of its positive effects over the glycemic index. There are lots of disadvantages about aspartame and it has a limited usage if you have to use it every day and also there are several incompatibilities reported in literature and safety problems. Thus, sucralose has an important role in this aspect and even if it is used in low amounts it has a synergistic taste improvement with mannitol which is also very important issue in orally disintegrating tablet formulations. According to this object of the present invention sucralose is present in an amount of between 0.01 to 2.0 % by weight, preferably it is 0.20 % by weight of total formulation.

Suitable flavouring agents may comprise but not limited to fruit flavours such as orange, banana, strawberry, cherry, wild cherry, lemon; and other flavours such as cardamom, anise, peppermint, menthol, vanillin and ethyl vanillin and the like and mixtures thereof; preferably the flavouring agent is fruit flavour such as orange in an amount of 1.0% by weight of total composition.

In a further aspect, the present invention shows that it is possible to have a significant influence on the disintegration rate of the tablet by modifying the dimensions and shape of the tablet. In general, as the tablet becomes thinner and have higher porosity, the orally disintegrating composition will be weakened faster when it contacts with saliva, because the disintegration process is produced after wetting all the surface of the tablet via capillary action. Also, any shape which maximizes the contact surface with the saliva may produce a significant reduction in disintegration time. The preferred shape of the orally disintegrating tablet composition of this invention may have a shape of a disk, circle, round, sphere, polygon, ellipse and the like. The preffered shape of the tablet is round/ellipse.

As it is mentioned above, to develop orally disintegrating compositions are difficult because of several different reasons. A satisfied orally disintegrating dosage form needs to meet number of requirements. In order to be pharmacologically acceptable, orally disintegrating compositions must be palatable. In addition, the orally disintegrating formulations must also provide acceptable pharmacokinetics and bioavailability to provide the desired therapeutic effect. Conversely, components of the formulation that promote rapid release may result in undesirable taste. Finally, any orally disintegrating composition with suitable organoleptic and pharmacokinetic properties must also be manufactured at commercially useful rates and yields. To fulfill all these requirements the formulation for a specific drug needs to be adapted in particular by a careful selection of the excipients used. However, the excipients selected may lead to formulations which are not bioavailable to the corresponding conventional dosage forms. Thus, they have to be chosen very carefully.

The present invention is further described by referring to the following example. Although the examples iare not intended to limit the scope of the present invention, it should be considered in the light of the description detailed above.

### Example 1

### Orally disitengrating flurbiprofen tablets

In this present invention, to minimize the disintegration time and maximise the mechanical resistance of the tablets of this invention, this orally disintegrating tablet formulation of flurbiprofen or a pharmaceutically acceptable salt thereof, according to the process described below has been designed consisting of;
a) 25.0 % by weight flurbiprofen sodium
b) 2.0 % polyvinylpyrrolidone
c) 2.0 % polyvinylalcohol
d) 1.50 % sodium stearyl fumarate
e) 15.0 % crospovidone
f) 30.3 % mannitol
g) 18.0 % microcrystalline cellulose
h) 5.0 % mixture of microcrystalline cellulose and guar gum
i) 1.0 % orange flavor
j) 0.20 % sucralose

According to main object of the invention a simple and time-saving process for the preparation of orally disintegrating tablet formulation of flurbiprofen or a pharmaceutically acceptable salt thereof is described below. Although the example is not intended to limit the scope of the present invention, it should be considered in the light of the description detailed above.

### Example 2

### Process for preparing the orally disintegrating flurbiprofen tablets

Flurbiprofen sodium is blended with 2.0 % by weight of polyvinylpyrrolidone and the mixture is granulated with alcohol and/or water. These granules are dried in 55°C and sieved by 1 mm. Sieved granules are coated with surfactant free mixture which is 2.0 % by weight polyvinyl alcohol. Then other excipients which are crospovidone, mannitol, microcrystalline cellulose, mixture of microcrystalline cellulose and guar gum, sucralose and orange flavor are added to this coated granule and mixed. Then sodium stearyl fumarate is added to this mixture and blended until obtaining a homogenous powder mixture. This homogeneous powder mixture is compressed to form tablets.

## Claims

1. A process for preparing orally disintegrating formulations of flurbiprofen or a pharmaceutically acceptable salt therof comprising the following steps;
a) blending 0.10 % to 5.0 % by weight of at least one polymeric binder which is selected from group consisting of polyvinylpyrrolidone, hydroxypropyl methylcellulose, hydroxypropyl cellulose, hydroxyethyl cellulose, methyl cellulose, ethyl cellulose, cellulose acetate, cellulose acetate phthalate, cellulose acetate trimellitate, hydroxypropyl methylcellulose phthalate, carboxymethylcellulose sodium, polyvinyl acetate, polyvinylacetate phthalate and vinylacetate crotonic acid copolymer and combinations thereof with flurbiprofen or a pharmaceutically acceptable salt therof,
b) granulating the mixture with alcohol and/or water,
c) coating the granule with a surfactant free mixture which is selected from a group comprising of polyvinyl alcohol or polyvinyl alcohol copolymers in a weight ratio between 0.50 to 5.0 % by weight,
d) adding other excipients to coated granule and mixing them,
e) adding sodium stearyl fumarate to this mixture and blending them until obtaining a homogenous powder mixture.

2. The process according to claim 1, wherein the polymeric binder is polyvinylpyrrolidone.

3. The process according to claim 1 and 2, wherein the weight ratio of polyvinylpyrrolidone is 2.0 % by weight.

4. The process according to claim 1, wherein the weight ratio of the surfactant free mixture of polyvinyl alcohol or polyvinyl alcohol copolymers is 2.0 % by weight.

5. The process according to claim 1, wherein the weight ratio of the sodium stearyl fumarate is between 0.10 to 5.0 %, preferably is between 0.50 to 3.0 % by weight, more preferably it is 1.50 % by weight.

6. The process according to claim 1, wherein the pharmaceutical acceptable salt of flurbiprofen is sodium salt.

7. The process according to claim 1, wherein the other excipients are selected from the group comprising of crospovidone, mannitol, microcrystalline cellulose, mixture of microcrystalline cellulose and guar gum, sucralose and orange flavor.

8. The process for preparing orally disintegrating formulations of flurbiprofen or a pharmaceutically acceptable salt therof according to any preceding claim comprising the following steps;
a) blending 0,01 % to 5,0 % by weight with polyvinylpyrrolidone with flurbiprofen sodium salt,
b) granulating the mixture with alcohol and/or water,
c) drying and sieving the granule,
d) coating the sieved granule with a surfactant free mixture which is polyvinyl alcohol or polyvinyl alcohol copolymers in a weight ratio between 2.0 % by weight,
e) adding crospovidone, mannitol, microcrystalline cellulose, mixture of microcrystalline cellulose and guar gum, sucralose and orange flavor to coated granule and mixing them,
f) adding sodium stearyl fumarate to this mixture and blending them until obtaining a homogenous powder mixture,
g) compressing the blended mixture to form tablets.

9. The process according to claim 8, wherein the weight ratio of crospovidone and sodium stearyl fumarate is between 25:1 to 1:1 by weight of the total composition, preferably between 15:1 to 1:1 (w/w), more preferably it is 10:1 (w/w).

10. The process according to claim 8, wherein the weight ratio of mannitol to microcrystalline cellulose is in the range of between 10:1 to 1:10 (w/w), preferably it is 5:1 to 1:5 (w/w), more preferably it is 2:1 to 1:2 (w/w).

11. Orally disintegrating tablet formulation of flurbiprofen or a pharmaceutically acceptable salt thereof according to claims 1 to 10, consisting of;
a) 25.0 % by weight flurbiprofen pharmaceutically acceptable salt thereof
b) 2.0 % polyvinylpyrrolidone
c) 2.0 % polyvinylalcohol
d) 1.50 % sodium stearyl fumarate
e) 15.0 % crospovidone
f) 30.3 %mannitol
g) 18.0 % microcrystalline cellulose
h) 5.0 % mixture of microcrystalline cellulose and guar gum
i) 1.0 % orange flavor
j) 0.20 % sucralose

12. Orally disintegrating tablet formulation according to claim 11, wherein the pharmaceutically acceptable salt of flurbiprofen is sodium salt.

13. Orally disintegrating tablet formulation according to claim 11 and 12, wherein the tablets disintegrate in oral cavity in less than 60 seconds, preferably in less than 30 seconds.

14. Orally disintegrating tablet formulation according to claim 11 and 12, wherein the hardness of the tablets are between 5 N to 100 N, preferably it is between 20 N to 50 N.

15. Orally disintegrating tablet formulation according to claim 11 and 12, wherein the friability of the tablets are less than 1.0 %, preferably less than 0.50 %.
